(19) Europäisches Patentamt
European Patent Office
Office européen des brevets

(11) EP 1 265 653 B1

(12) EUROPEAN PATENT SPECIFICATION

(45) Date of publication and mention of the grant of the patent:
**02.06.2004 Bulletin 2004/23**

(21) Application number: **01915375.8**

(22) Date of filing: **19.03.2001**

(51) Int Cl.[7]: **A61L 33/06**, C08G 79/02, A61L 27/34, A61L 29/08, A61L 31/10, A61K 6/08, A61P 31/04

(86) International application number:
**PCT/EP2001/003108**

(87) International publication number:
**WO 2001/070296 (27.09.2001 Gazette 2001/39)**

# (54) USE OF POLYPHOSPHAZENE DERIVATIVES FOR ANTIBACTERIAL COATINGS

VERWENDUNG VON POLYPHOSPHAZEN-DERIVATEN FÜR ANTIBAKTERIELLE BESCHICHTUNGEN

USAGE DE DERIVES DE POLYPHOSPHAZENE POUR DES REVETEMENTS ANTIBACTERIENS

(84) Designated Contracting States:
**AT BE CH CY DE DK ES FI FR GB GR IE IT LI LU MC NL PT SE TR**

(30) Priority: **18.03.2000 DE 10013639**

(43) Date of publication of application:
**18.12.2002 Bulletin 2002/51**

(60) Divisional application:
**03003153.8 / 1 312 635**

(73) Proprietor: **Polyzenix GmbH**
**76137 Karlsruhe (DE)**

(72) Inventor: **GRUNZE, Michael**
**69151 Neckargemünd (DE)**

(74) Representative: **Müller-Boré & Partner Patentanwälte**
**Grafinger Strasse 2**
**81671 München (DE)**

(56) References cited:

| | |
|---|---|
| **WO-A-99/16477** | **DE-A- 19 613 048** |
| **US-A- 4 451 647** | **US-A- 4 880 622** |
| **US-A- 5 548 060** | **US-A- 6 077 916** |

- **ALLCOCK: "POLYPHOSPHAZENES" INORGANIC POLYMERS, 1992, pages 61-139, XP000866367**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

The present invention relates to the use of polyphosphazene derivatives, having excellent biocompatible properties and imparting bacterial resistance to a coating of an article such as a medical device. In particular, the coating is applied on at least part of a surface of e.g. said medical device and can be used for preventing and/or reducing an inflammatory response upon application of said medical device to a patient.

The major complications from artificial temporary or permanent implants and catheters are, on the one hand, increased deposition of thrombocytes at the surface of the foreign body. On the other side, the behaviour towards bacterias, macrophages and proteins deposited on the surface of the implants plays an important role, since those deposits essentially lead to inflammations and other problems concerned when the implants are growing in.

One of the problems occurring is, for example, increased cell proliferation and inflammation of the injured tissue that comes into contact with the artificial implant. With vascular implants, such as "stents", there are not only the well-known problems of increased thrombus formation, but also restenoses (i.e. re-narrowing of the blood vessel in the region expanded by angioplasty, frequently the stent region). Those complications are initiated because of activation of the clotting and immune system by the implanted foreign object, and by damage to the vessel wall during implantation of the stent in the course of angioplasty. As a consequence, the so-called restenosis (re-narrowing of the blood vessel) is caused, and, possibly, inflammations in the treated region, so that medical and chirurgical treatment is needed promptly.

One possibility having been used to deal with those complications to prevent excessive cell proliferation, is the use of so-called covered and coated stents. A plurality of materials and also covered or coated stents usable for the preparation of such coverings or coatings are known in the prior art and have been studied. For example, WO 98/56312 describes an expandable covering made of ε-PTFE for that purpose. Other materials for that use are described in EP-A-0 810 845 in which polymers described in US-A 4,883,699 and US 4,911,691 are mentioned. Further polymers given for that purpose are, for example, hydrolyzed polyacrylonitrile (US-A 4,480,642), hydrophilic polyethers (US-A-4,798,876), and polyurethane-di-acrylates (US-A-4,424,395). Further, various hydrogels usable for that purpose are known. The number of potentially usable materials further includes poly(vinylpyrrolidone) (PVP) polymers, poly (vinyl alcohols) (PVA), poly(ethylene oxide) polymers (PEO) and poly(hydroxyethyl methacrylate) p(HEMA). Further, there are publications describing the application of a number of standard materials like polyurethanes, polyethylenes, and polypropylenes as possible materials. Mixtures of these materials are also known. A number of further materials is known from EP-A-0 804 909.

WO 99/16477 describes polyphosphazene derivatives having a radioactive component, e.g. radioactive phosphorus isotope, and the use thereof to provide antithrombogenic coating of implants. US-A-4,451,647 describes heparinized polyphosphazene coatings of medical articles, e.g. catheters, blood bags or dialysis membranes. US-A-4,880,622 describes the use of polyphosphazene derivatives for controlled drug release or as antifoaming agent. US-A-5,548,060 describes a method for the alkylsulfonation of phosphazene for use as biomaterials. Allcock ("Polyphosphazenes" in Inorganic Polymers, 1992, 61-139) describes polyphosphazenes derivatized with amino, alkoxy or sulfonic acid groups for improving blood compatibility.

The above compounds have different properties. It can be supposed that each of the materials has specific properties for certain applications. For example, PVA dissolves in liquids very well. Other materials exhibit good blood tolerance. On the other hand, some materials are particularly well extendable. Unfortunately, however, all materials have deficiencies in certain areas. For example, PVA does not exhibit good blood tolerance. ε-PTFE can be extended very well, for example, and has also good blood tolerance, but is difficult to handle. The preparation of such coverings and coatings requires a number of processing steps (WO 96/00103). Some other materials can only be made elastically by addition of plasticizers, which reduce the blood tolerance and physical tolerance and represent a further distress for the patient due to the "flushing" of the plasticizer.

Therefore, due to the unsufficient properties of the presently available materials, at present, patients are given clotting inhibitors (Vitamin K antagonists) during the postoperative treatment following angioplasty; but the dosages are problematical and show no effect concerning the inhibition of inflammation or an auto-immune response, as well as an inhibitory effect concerning restenosis.

The frequency of restenosis for the usual commercially available stents is about 30-50% within 6 months after successful angioplasty.

When using coated stents which help to avoid a reaction and especially the activation of an inflammatory response, the rate of restenoses should be decreased by preventing the cell tissue to grow into the blood vessel region. However, that technique is limited particularly by the materials and their physical and chemical properties as well as their surface properties (surface finish).

The polymeric compound poly[bis(trifluoroethoxy)phosphazene] exhibits good antithrombogenic action as a filler (see Tur, Untersuchungen zur Thrombenresistenz von Poly[bis(trifluoroethoxy)phosphazen] [Studies of resistance of poly[bis(trifluoroethoxy)phosphazene] to thrombus formation] and Hollemann Wiberg, "Stickstoffverbindungen des

Phosphors" [Nitrogen compounds of phosphorus], Lehrbuch der anorganischen Chemie [Textbook of Inorganic Chemistry], 666-669, 91st-100th Edition, Walter de Gruyter Verlag, 1985; and Tur, Vinogradova et al., "Entwicklungstendenzen bei polymeranalogen Umsetzungen von Polyphosphazenen" [Trends in development of polymer-like reactions of polyphosphazenes], Acta Polymerica 39, No. 8, 424-429 (1988)). Further, polyphosphazene is used in German Patent 196 13 048 for coating artificial implants, with the intention to avoid thrombus formation on the surface of the implants, and especially heart walls can be ameliorated by this type of coating.

Of course, there remains the risk of inflammations as usual reaction in response to the incorporation of e.g. artificial implants into a patient, which can be only minimized by application of e.g. antibiotics. However, the severe disadvantage of bacterial multiple resistance when administering regularly antibiotics as a precaution to prevent an inflammation response is well known and is becoming more and more important. Nevertheless, even the use of antibiotics can not avoid certain auto-immune reactions caused by the devices used.

Therefore, the technical problem underlying the present invention is to provide a new system and method for e.g. medical devices, which should, on one hand, impart outstanding mechanical characteristics and physically tolerated properties to the medical devices so as to improve the biocompatibility of the medical devices, and should also prevent or reduce the previously mentioned sequelae of successful treatment or implantation, arid on the other hand, should exhibit, besides antithrombogenic properties, the effect of preventing or reducing inflammations and auto-immune reactions as a reaction in response to the incorporation of the foreign body into the organism, in order to reduce the dosage of or even to avoid the administration of antibiotics and to ameliorate the acceptance of a foreign device coming into contact with body tissue.

The above problem is solved by the use of a polymer having the following general formula (I)

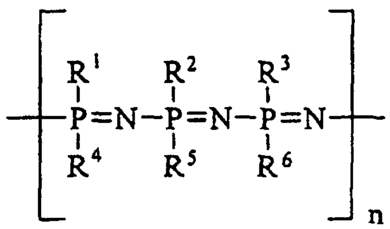

(I)

wherein

n is from 2 to ∞,

$R^1$ to $R^6$ are the same or different and represent an alkoxy, alkylsulfonyl, dialkylamino or aryloxy group, or a heterocycloalkyl or heteroaryl group in which nitrogen is the heteroatom,

for imparting bacterial resistance to a surface. In a preferred embodiment, the polymer is a biocompatible polymer which can be used for imparting bacterial resistance to a coating. The term "bacterial resistance" encompasses a passivating coating or surface against bacterial adhesion and/or proliferation.

The coating can be applied to any article. The term "article" encompasses any article without any particular limitation of the form or shape, but with the need of asepsis or bacterial resistance. Examples of said article, but not limited to, include walls and furniture in hospitals, and, in a preferred embodiment, medical devices. The medical device having the above coating on at least part of a surface of said device, can be used for preventing and/or reducing an inflammatory response upon application of said medical device to a patient.

The term "medical device" encompasses any medically useable device, particularly devices which come into direct contact with tissue and/or body fluids of a patient. Examples of said medical device include artificial implants such as plastic implants for e.g. breast, nose or ear, bone nails, bone screws, bone plates, artificial (urinary) bladder, artificial cartilage, dental implants, artificial bones for e.g. artificial hip or hip joints, artificial esophagus and artificial trachea; artificial (arterial and veinous) blood vessels; stents such as urological stents and cardiovascular stents; catheters such as urological catheters and cardiovascular catheters; cardiovascular grafts; emplastrums; dermatoplastics; devices, e.g. in the gastrointestinal tract, in the prostata, in the urinary tract, or for the protection of neurons and neurofibers; therapeutic devices such as cardiac pacemakers, defibrillators, electrodes for cardiac pace- makers and defibrillators, surgical devices, surgical instruments, artificial biological membrans and artificial organs such as artificial kidneys and artificial heart.

The above defined polymer imparts not only biocompatibility to a coating for e.g. medical devices, but also anti-thrombogenic properties and bacterial resistance. Thus, substantially no thrombus formation, no autoimmune response and no inflammatory response of medical devices can be observed upon application to a patient. This surprising result is *inter alia* based on the fact that the blood components such as macrophages, and bacteria do not adhere and, in the case of bacteria, can not grow on the surface of said coating.

The degree of polymerization of the polymer used in the coating according to the present invention can be from 2 to ∞. However, the preferred range for the degree of polymerization is from 20 to 200,000, and more preferably 40 to 10,000,000.

Preferably, at least one of the groups $R^1$ to $R^6$ in the polymer used is an alkoxy group substituted with at least one fluorine atom.

The alkyl groups in the alkoxy, alkylsulfonyl and dialkylamino groups are, for example, straight or branched alkyl groups with 1 to 20 carbon atoms, in which the alkyl groups can, for example, be substituted with at least one halogen atom, such as a fluorine atom.

Examples of alkoxy groups are the methoxy, ethoxy, propoxy and butoxy groups, which can preferably be substituted with at least one fluorine atom. The 2,2,2-trifluoroethoxy group is particularly preferred. Examples of alkylsulfonyl groups are methylsulfonyl, ethylsulfonyl, propylsulfonyl and butylsulfonyl groups. Examples of dialkylamino groups are dimethylamino, diethylamino, dipropylamino and dibutylamino groups.

The aryl group in the aryloxy group is, for example, a compound with one or more aromatic ring systems, in which the aryl group can, for example, be substituted with at least one alkyl group as previously defined. Examples of aryloxy groups are the phenoxy and naphthoxy groups and their derivatives.

The heterocycloalkyl group is, for instance, a ring system containing 3 to 7 atoms, with at least one ring atom being a nitrogen atom. The heterocycloalkyl group can, for example, be substituted with at least one alkyl group as previously defined. Examples of heterocycloalkyl groups are the piperidinyl, piperazinyl, pyrrolidinyl and morpholinyl groups and their derivatives. The heteroaryl group is, for example, a compound with one or more aromatic ring systems in which at least one ring atom is a nitrogen atom. The heteroaryl group can, for example, be substituted with at least one alkyl group as previously defined. Examples of heteroaryl groups are the pyrrolyl, pyridinyl, pyridinolyl, isoquinolinyl and quinolinyl groups and their derivatives.

The biocompatible coating of the medical device or of the article according to the invention has, for example, a thickness of about 1 nm up to about 100 µm, preferably up to about 10 µm, and particularly preferably up to about 1 µm.

There is no particular limitation on the medical device or the article used as the substrate for the coating according to the invention and it can be any material, such as plastics, metals, metal alloys and ceramics, and the biocompatible polymer (as the matrix material or filler).

In one embodiment of the present invention, a layer containing an adhesion promoter is provided between the surface of the substrate and the biocompatible coating containing the polyphosphazene derivative of formula (I).

Preferably, the adhesion promoter or spacer, respectively, contains a polar end group. Examples are hydroxy groups, carboxy groups, carboxyl groups, amino groups or nitro groups, but end groups of the O-ED type can also be used, wherein O-ED represents an alkoxy, alkylsulfonyl, dialkylamino or aryloxy group, or a heterocycloalkyl or heteroaryl group in which nitrogen is the heteroatom, and can have different substitutents like halogen atoms, particularly fluorine atoms.

Particularly, the adhesion promoter is, for example, a silicium-organic compound, preferably an amino-terminated silane or based on aminosilane, amino-terminated alkenes, nitro-terminated alkenes and silanes, or an alkylphosphonic acid. Aminopropyltrimethoxysilane is particularly preferred.

The adhesion promoter particularly improves adhesion of the coating to the surface of an article such as a medical device by coupling the adhesion promoter to the surface of the medical device, for instance by ionic and/or covalent bonds and by further coupling of the adhesion promoter to reactive components, particularly to the above described polymer with the general formula (I) of the coating, for instance, through ionic and/or covalent bonds.

In one embodiment of the present invention, the medical device having the biocompatible coating exhibits surprisingly the adhesion and/or proliferation of specific eukaryotic cells on the surface of said medical device. For example, the medical device used as an artificial blood vessel shows the adhesion and growth of endothelial cells. Another example is the adhesion and growth of osteocytes on the surface of artificial bones having the biocompatible coating as defined above.

The following examples illustrate further the present invention, but should not be construed as limiting the scope of protection conferred by the claims.

**EXAMPLE 1**

A 0.1 M solution of polydichlorophosphazene (0.174 g per 5 ml absolute toluene as solvent) is prepared under an inert gas atmosphere. The artificial implant which was oxidatively purified, is put into this solution at room temperature for 24 h. Then, the thus immobilized polydichlorophosphazene on the artificial implant is esterified with 2,2,2-sodiumtrifluorethanolate in absolute tetrahydrofuran as solvent (8 ml absolute tetrahydrofuran, 0.23 g sodium, 1.56 ml 2,2,2-trifluorethanol). The reaction mixture is kept under the reflug for the whole reaction time. The esterification is carried out under an inert gas atmosphere at 80°C for a reaction time of 3 h. Then, the so coated substrate is washed with 4-5 ml absolute tetrahydrofuran and dried in a nitrogen stream.

After these treatments the surface was analyzed for elementary composition, stoichiometry and coating thickness using X-ray spectroscopy. The results show that all reaction steps have been successfully carried out, and that coating thicknesses of greater than 3.4 nm have been attained.

For a bacterial resistance test, the thus obtained implant is put in a petri disk which was filled with a suspension of E. coli in a nutrient solution. After 14 days incubation the artificial implant was taken off the suspension and analyzed microscopically. No adherence or growth of bacteria on the coated artificial implant could be detected.

**EXAMPLE 2**

The artificial implant which was oxidatively purified using Caro's acid, is immersed into an 2% solution of aminopropyltrimethoxysilane in absolute ethanol. Then, the substrate is washed with 4-5 ml absolute ethanol and kept at 105°C for 1 h in a dryer.

After coupling of aminopropyltrimethoxysilane on the oxidatively purified surface of the substrate the thus treated substrate is put into a 0.1 M solution of polydichlorophosphazene in absolute toluene for 24 h at room temperature under an inert gas atmosphere. Then, the so obtained artificial implant is treated as in Example 1, resulting in an artificial implant containing a coating with a thickness of < 5.5 nm.

The bacterial resistance test as described in Example 1 revealed that no adherence or growth of bacteria on the surface of the artificial implant could be detected.

**EXAMPLE 3**

Example 3 was carried out as described in Example 2 except that, after the coupling of aminopropyltrimethoxysilane, the artificial implant is put into a 0.1 M solution of poly[bis(trifluorethoxy)phosphazene] in ethylacetate (0.121 g per 5 ml ethylacetat) for 24 h at room temperature. Then, the thus prepared artificial implant is washed with 4-5 ml ethylacetat and dried in a nitrogen stream.

The X-ray spectroscopy revealed that the immobilisation of poly[bis(trifluorethoxy)polyphosphazene] via aminopropyltrimethoxysilan as an adhesion promoter has been successfully carried out and coating thicknesses of < 2.4 nm.

The bacterial resistance test as described in Example 1 revealed that no adherence or growth of bacteria on the surface of the artificial implant could be detected.

**EXAMPLE 4**

The artificial implant which was oxidatively purified with Caro's acid, is put into a 0.1 M solution of poly[bis (trifluorethoxy)phosphazene] in ethylacetate (0.121 g per 5 ml ethylacetate) at 70°C for 24 h. Then, the thus ,treated artificial implant is washed with 4-5 ml ethylacetate and dried in a nitrogen stream.

The analysis shows that the coupling of poly[bis(trifluorethoxy)phosphazene] is successfully carried out on the surface of the implant, and that coating thicknesses of more < 2.1 nm have been attained.

The bacterial resistance test as described in Example 1 revealed that no adherents or growth of bacteria on the surface of the artificial implant could be detected.

**EXAMPLE 5**

The artificial implant which was oxidatively purified with Caro's acid, is put into a melt of poly[bis(trifluorethoxy) phosphazene] at 70°C and kept for about 10 sec. up to about 10 h. Then, the thus treated artificial implant is washed with 4-5 ml ethylacetate and dried in a nitrogen stream.

The analysis shows that the coupling of poly[bis(trifluorethoxy)phosphazene] is successfully carried out on the surface of the implant, and. that coating thicknesses of up to several millimeters have been attained.

The bacterial resistance test as described in Example 1 revealed that no adherents or growth of bacteria on the surface of the artificial implant could be detected.

The articles such as medical devices containing the above defined coating and used according to the present invention surprisingly maintain the outstanding mechanical properties of the material of e.g. the artificial implants. Therefore, not only the biocompatibility of e.g. such medical devices can be drastically improved, but also the antithrombogenic properties can be drastically improved together with minimizing the risk of an inflammation upon application of the medical device to a patient due to the bacterial resistance of the coating.

Moreover, the coating applied increases the chemical and physical resistance of the medical device, which improves drastically e.g. the usage of stents to be applied in the urology, since no depositing of salts can be observed, which does not allow adhesion of and subsequently growth of bacteria. Thus, the risk of inflammation is reduced. Further, corrosion resistance of medical devices such as stents having the biocompatible coating, can be improved.

## Claims

1. Use of a polymer having the following general formula (I)

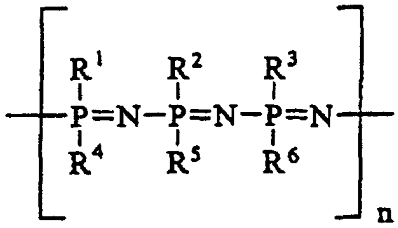

(I)

wherein

| | |
|---|---|
| n | is from 2 to $\infty$; |
| $R^1$ to $R^6$ | are the same or different and represent an alkoxy, alkylsulfonyl, dialkylamino or aryloxy group, or a heterocycloalkyl or heteroaryl group in which nitrogen is the heteroatom, |

for imparting bacterial resistance to a coating.

2. The use of a polymer according to claim 1 for the manufacture of a medical device comprising a coating which is applied on at least part of a surface of the medical device, wherein said coating contains the polymer as defined in claim 1, for preventing and/or reducing an inflammatory response upon application to a patient.

3. Use according to Claim 2, wherein the medical device is selected from the group consisting of artificial implants, emplastrums, artificial blood vessels, stents, catheters, plastic implants, bone nails, bone screws, bone plates, artificial bladder, artificial cartilage, dental implants, artificial bones, artificial esophagus, artificial trachea, and therapeutic devices.

4. The use according to Claim 3, wherein artificial implants include plastic implants, bone nails, bone screws, bone plates, artificial bladder, artificial cartilage, dental implants, artificial bones, artificial esophagus, and artificial trachea.

5. The use according to Claim 3, wherein artificial blood vessels comprise both arterial and veinous vessels.

6. The use according to Claim 3, wherein the stents include urological stents and cardiovascular stents.

7. The use according to Claim 3, wherein the catheters include urological catheters and cardiovascular catheters.

8. The use according to Claim 3, wherein the therapeutic devices include cardiac pacemakers, defibrillators, electrodes for cardiac pacemakers and defibrillators, and surgical devices.

## Patentansprüche

1. Verwendung eines Polymers, das die folgende allgemeine Formel (I) aufweist

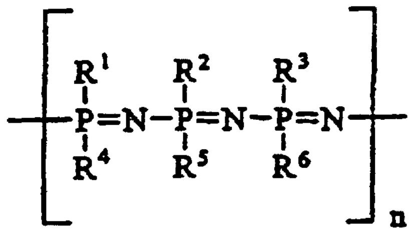

(I)

wobei

n von 2 bis ∞ ist,

$R^1$ bis $R^6$ gleich oder verschieden sind und eine Alkoxy-, Alkylsulfonyl-, Dialkylamino- oder Aryloxygruppe oder eine Heterocycloalkyl- oder Heteroarylgruppe, in der das Heteroatom Stickstoff ist, darstellen,

um einem Überzug eine Bakterienresistenz zu verleihen.

2. Verwendung eines Polymers gemäß Anspruch 1 zur Herstellung einer medizinischen Vorrichtung, umfassend einen Überzug, der auf mindestens einen Teil einer Oberfläche der medizinischen Vorrichtung aufgetragen ist, wobei der Überzug das wie in Anspruch 1 definierte Polymer enthält, zum Verhindern und/oder Reduzieren einer Entzündungsreaktion bei Anwendung bei einem Patienten.

3. Verwendung gemäß Anspruch 2, wobei die medizinische Vorrichtung aus der Gruppe, bestehend aus künstlichen Implantaten, Emplastren, künstlichen Blutgefäßen, Stents, Kathetern, plastischen Implantaten, Knochennägeln, Knochenschrauben, Knochenplatten, einer künstlichen Blase, künstlichem Knorpel, dentalen Implantaten, künstlichen Knochen, einer künstlichen Speiseröhre, einer künstlichen Luftröhre und therapeutischen Vorrichtungen, ausgewählt ist.

4. Verwendung gemäß Anspruch 3, wobei die künstlichen Implantate plastische Implantate, Knochennägel, Knochenschrauben, Knochenplatten, eine künstliche Blase, künstlichen Knorpel, dentale Implantate, künstliche Knochen, eine künstliche Speiseröhre und eine künstliche Luftröhre einschließen.

5. Verwendung gemäß Anspruch 3, wobei die künstlichen Blutgefäße sowohl arterielle als auch venöse Gefäße umfassen.

6. Verwendung gemäß Anspruch 3, wobei die Stents urologische Stents und kardiovaskuläre Stents einschließen.

7. Verwendung gemäß Anspruch 3, wobei die Katheter urologische Katheter und kardiovaskuläre Katheter einschließen.

8. Verwendung gemäß Anspruch 3, wobei die therapeutischen Vorrichtungen Herzschrittmacher, Defibrillatoren, Elektroden für Herzschrittmacher und Defibrillatoren und chirurgische Vorrichtungen einschließen.

**Revendications**

1. Utilisation d'un polymère répondant à la formule 'générale (I) suivante

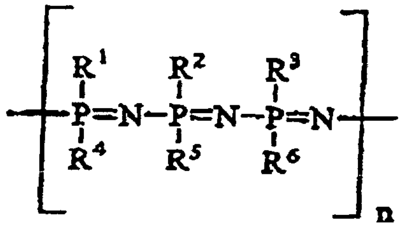

(I)

dans laquelle

n a une valeur de 2 à ∞ ;

$R^1$ à $R^6$ sont identiques ou différents et représentent un groupe alkoxy, alkylsulfonyle, dialkylamino ou aryloxy, ou un groupe hétérocycloalkyle ou hétéroaryle dans lequel l'hétéroatome est un atome d'azote,

pour conférer une résistance aux bactéries à un revêtement.

**2.** Utilisation d'un polymère suivant la revendication 1, pour la production d'un dispositif médical comprenant un revêtement qui est appliqué sur au moins une partie d'une surface du dispositif médical, dans laquelle ledit revêtement contient le polymère répondant à la définition suivant la revendication 1, pour la prévention et/ou la réduction d'une réponse inflammatoire lors de l'application à un patient.

**3.** Utilisation suivant la revendication 2, dans laquelle le dispositif médical est choisi dans le groupe consistant en des implants artificiels, des emplâtres, des vaisseaux sanguins artificiels, des extenseurs, des cathéters, des implants en matière plastique, des clous pour les os, des vis pour les os, des plâtres pour les os, une vessie artificielle, un cartilage artificiel, des implants dentaires, des os artificiels, un oesophage artificiel, une trachée artificielle et des dispositifs thérapeutiques.

**4.** Utilisation suivant la revendication 3, dans laquelle les implants artificiels comprennent des implants en matière plastique, des clous pour les os, des vis pour les os, des plâtres pour les os, une vessie artificielle, un cartilage artificiel, des implants dentaires, des os artificiels, un oesophage artificiel, une trachée artificielle.

**5.** Utilisation suivant la revendication 3, dans laquelle les vaisseaux sanguins artificiels comprennent des vaisseaux artificiels et des vaisseaux veineux.

**6.** Utilisation suivant la revendication 3, dans laquelle les extenseurs comprennent des extenseurs urologiques et des extenseurs cardiovasculaires.

**7.** Utilisation suivant la revendication 3, dans laquelle les cathéters comprennent des cathéters urologiques et des cathéters cardiovasculaires.

**8.** Utilisation suivant la revendication 3, dans laquelle les dispositifs thérapeutiques comprennent des stimulateurs cardiaques, des défibrillateurs, des électrodes pour les stimulateurs cardiaques et les défibrillateurs cardiaques, ainsi que des dispositifs chirurgicaux.